# EUROPÄISCHE PATENTSCHRIFT

(11) **EP 4 171 430 B1**
(45) Veröffentlichungstag und Bekanntmachung des Hinweises auf die Patenterteilung: **15.04.2026**
(21) Anmeldenummer: 21737403.2
(22) Anmeldetag: 25.06.2021
(51) Int. Cl.: A61B 46/10, A61B 17/00, H01R 13/52

(54) **ELEKTRO-MECHANISCHE STERILTRENNUNG**
ELECTROMECHANICAL STERILE SEPARATION
SÉPARATION ÉLECTROMÉCANIQUE STÉRILE

(30) Priorität: 25.06.2020 DE 102020116769
(43) Veröffentlichungstag der Anmeldung: 03.05.2023
(73) Patentinhaber: Aesculap AG, 78532 Tuttlingen (DE)
(72) Erfinder: HÖGERLE, Roland-Alois, 78532 Tuttlingen (DE); KETTERER, Thorsten, 78464 Konstanz (DE)
(74) Vertreter: Winter, Brandl - Partnerschaft mbB
(86) Internationale Anmeldenummer: PCT/EP2021/067533
(87) Internationale Veröffentlichungsnummer: WO 2021/260191

(56) Entgegenhaltungen:
- WO-A1-2016/134266
- DE-A1- 102015 207 953
- US-A1- 2018 145 443
- US-A1- 2020 069 383
- US-A1- 2020 170 724

## Beschreibung

Die Offenbarung betrifft eine Sterilbarriere mit einem Abdeck- oder Abtrennelement, z.B. eine Plane oder Folie (auch als Drape bezeichnet) und einer darin integrierten oder integrierbaren/montierbaren elektro-mechanischen, keimdichten Schleuse zur Übertragung von elektrischer Energie und/oder elektrischen Steuersignalen von einer unsteril-seitigen Verkabelung auf eine steril-seitige Verkabelung und insbesondere eine Sterilbarriere nach dem Oberbegriff des Patentanspruchs 1.

### Stand der Technik

Zunehmende Digitalisierung im OP führt zu einer zunehmenden Integration von Elektronik und Software in elektrisch angetriebenen chirurgischen Motorensystemen. Da elektronische Elemente nur mit großem Aufwand und nicht uneingeschränkt aufbereitbar sind, wird grundsätzlich angestrebt, beispielsweise chirurgische Antriebe in einem sterilen Bereich (Materialien uneingeschränkt aufbereitbar und vom Chirurgen uneingeschränkt bedienbar) und in einem unsterilen Bereich (aufbereitungskritische Materialien und vom Chirurgen nicht berührbar) anzubieten bzw. aufzuteilen. Ein Vorteil dieses Konzepts ist, dass der Chirurg die kleinstmögliche Masse (Volumen) handhaben muss, da wesentliche Teile der chirurgischen Antriebe in den unsterilen Bereich ausgelagert sind. Diese beiden Bereiche (steriler Bereich / unsteriler Bereich) müssen dann einerseits vor der Operation elektromechanisch kontaktiert und andererseits während der OP wechselweise dekontaktiert / getrennt und mit anderen Sterilbereichen wieder kontaktiert werden. Idealerweise sind diese Arbeiten vom sterilen Personal am Operationstisch, unter Einhaltung von Sterilanforderungen durchzuführen. Dies erfordert ein den aktuellen Anforderungen an eine Operationshygiene angepasstes und sicher bedienbares Steriltrennungskonzept.

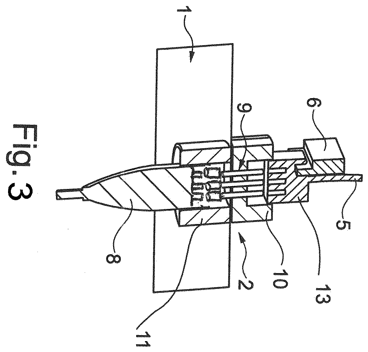

Die anliegende Fig. 3 zeigt eine allgemeine Anordnung eines Steril- und Unsterilbereichs, die durch eine Sterilbarriere getrennt sind und wie es auch für die vorliegende Offenbarung vorgesehen sein kann.

Beispielhaft befindet sich eine Robotik 100 einschließlich Stellantriebe oder eine Haltevorrichtung in einem Unsterilbereich U, der mittels eines Drape 101 vom umgebenden Sterilbereich S abgeschirmt ist. Das Drape hat wenigstens zwei Schleusen/Öffnungen 102, 103, wovon die eine Schleuse 102 als Durchtrittsöffnung für einen Haltearm der Robotik 100 und die andere Schleuse 103 zum Konnetieren eines sterilen Kabelabschnitts 104 im Sterilbereich S mit einem unsterilen Kabelabschnitt innerhalb des Unsterilbereichs U (nicht gezeigt) ausgebildet ist. Hierfür ist eine Konnektorvorrichtung 105 vorgesehen. In diesem Fall ist das Kabel im Sterilbereich S an eine Steuerung 106 beispielsweise zur Ansteuerung der Robotik 100 angeschlossen.

Grundsätzliche weitere Anwendungsbeispiele sind z. B. eine Kontaktierung eines Antriebsmotors im Sterilbereich an einem Steuermodul oder einem Roboterarm im Unsterilbereich und dergleichen..

Bereits weiter bekannt ist, z. B. auf einem sterilen Motorkabel einen so genannten Schutzschild anzubringen. Der Schutzschild ist so positioniert, dass er die Gefahr der Berührung unsteriler Komponenten während eines Konnektions-/Steckvorgangs bzw. des Steckens des Motorkabels ins unsterile Steuergerät reduziert. Dieser sogenannte Schutzschild wird auch Sterilschutzkappe genannt.

Ein wesentlicher Nachteil daran ist jedoch, dass nach einmaligem Konnektieren/Stecken das Motorkabel zumindest im Bereich seines Konnektors kontaminiert ist und ein Entkoppeln/Abstecken des Kabels und Zurücklegen auf einen sterilen Instrumententisch innerhalb des Sterilbereichs nicht mehr möglich ist. Dies verhindert, dass während einer Operation verschiedene Antriebe für verschiedene Operationsschritte genutzt werden können.

Des Weiteren existieren im Stand der Technik sogenannte Sterilbarrieren in Form von Abdeck- oder Abtrennplanen, welche den Sterilbereich vom Unsterilbereich trennen und mit einem Interface bzw. einer Schnittstelle ausgerüstet sind, über welches Versorgungsleitungen hindurchgeführt werden können. Hierfür hat ein solches Interface eine Anzahl von Klappen, die geöffnet und geschlossen werden können, um so entsprechende Durchgänge wahlweise zu schaffen.

US 2018/0 145 443 A1 offenbart einen elektrischen Verbinder, der einer innerhalb eines Körpers eines Patienten bewegbaren und nachverfolgbaren elektromagnetischen Nachführeinrichtung zugeordnet ist. Dabei ist in einem mehrteiligen elektrisches (Steck-)Verbindersystem, das in einer medizinischen Umgebung verwendet wird, ein erster Abschnitt des Verbindersystems so angeordnet, dass er eine formbare Sterilbarriere, bspw. aus einem faserigen Material, durchstößt, wobei dies unter Kopplung eines ersten Teils des Verbindersystems an einen zweiten Teil des Verbindersystems erfolgt. Jedoch ist hierbei nachteilig, wie insbesondere anhand der Figuren 9, 11A sowie 11E US 2018/0 145 443 A1 nachzuvollziehen, dass mit dem vorgeschlagenen Durchstoßen der Weg für Keime an der betreffenden Stelle (lokal) frei ist. Infolge einer ungehinderten Verkeimung über das durchgestoßene Loch hinweg treten naturgemäß eine Beeinträchtigung bzw. ein Aufheben einer zuvor ggf. gegebenen Sterilität ein.

US 2018/0 049 833 A1 offenbart eine sterile Barriere zur Verwendung mit einem chirurgischen Robotersystem. Die sterile Barriere kann ein Gehäuse umfassen, das konfiguriert ist, um mit einem Werkzeugfahrer zusammenzupassen, der mit einem Roboterarm verbunden ist, und um ein chirurgisches Werkzeug entfernbar aufzunehmen. Das Gehäuse der sterilen Barriere weist ein steriles flexibles Gewebe auf, das sich davon erstreckt, so dass das Gehäuse und das flexible Gewebe in mehr als einer Ebene ausgebildet sind und eine sterile Seite definieren, auf der das chirurgische Werkzeug angeordnet ist, und eine nicht-sterile Seite, auf der das Werkzeug angeordnet ist Das Gehäuse kann zumindest einen elektrischen stiftförmigen Kontakt aufweisen, der so konfiguriert ist, dass er funktionsfähig mit mindestens einem komplementären elektrischen Kontakt an mindestens einem der Werkzeugfahrer und des Roboterarms zusammenpasst. Dabei ist das Gewebe mit vorgefertigten Öffnungen ausgebildet, die angeordnet bzw. eingerichtet sind, um die elektrischen stiftförmigen Kontakte durch diese jeweilig hindurchzuführen.

US 2016/0 058 513 A1 offenbart eine weitere im Stand der Technik bekannte Sterilbarriere. Dabei umfasst ein chirurgisches System ein erstes Element mit einem Sitz, der entfernbar ein chirurgisches Instrument zur betrieblichen Verwendung davon aufnimmt. Mindestens einer von zwei Verbindern, der mit elektrischen Kontakten zum Eingreifen in den anderen Verbinder versehen ist, ist so ausgelegt, dass er steuerbar zwischen einer ersten zurückgezogenen Ruheposition und einer zweiten vorgerückten Position für die elektrische Verbindung zusammen mit dem anderen Verbinder beweglich ist.

Ferner offenbart DE 20 2007 002 332 U1 der vorliegenden Anmelderin einen medizinischen Sterilüberzug, wobei ein Steckkontakt durch jeweilige in der Hülle vorgesehene Öffnungen in die jeweilige Steckbuchse eingeführt wird. Dabei werden zum Zwecke der Abdichtung feste Verklebungen vorgeschlagen.

WO 2016/134266 A1 offenbart eine Sterilbarriere-Anordnung, ein Befestigungssystem und ein Verfahren zum kinematischen Koppeln von ersten und zweiten chirurgischen Komponenten durch die Sterilbarriere, so dass die Positionierung wiederholbar und deterministisch ist.

US 2020/170724 A1 offenbart ein (robotisches) Befestigungssystem zum Verbinden von ersten und zweiten chirurgischen Komponenten. Das Befestigungssystem umfasst einen ersten und einen zweiten Befestigungsabschnitt, und einen Spanner, der zwischen einer ersten Position und einer zweiten Position beweglich ist, und eine sterile Barriereanordnung. Die sterile Barriereanordnung umfasst eine Kupplung, die konfiguriert ist, um lösbar an dem ersten Befestigungsabschnitt befestigt zu sein und den zweiten Befestigungsabschnitt lösbar aufzunehmen, wenn der Spanner des zweiten Montageteils in der ersten (offenen) Position ist, und eine Vielzahl von kinematischen Kupplungen, die konfiguriert sind, um die Befestigungsabschnitte in Eingriff zu bringen, und die angeordnet sind, um eine kinematische Kopplung zwischen den Befestigungsabschnitten durch die sterile Barriereanordnung zu schaffen, um sechs Freiheitsgrade der Bewegung zwischen den chirurgischen Komponenten einzuschränken, wenn sich der Spanner des zweiten Befestigungsabschnitts in der zweiten (Halte-) Position befindet.

DE 10 2015 207953 A1 offenbart eine besonders robuste Ausführung einer elektrischen Steckerverbindung außerhalb des medizinischen Bereiches.

US 2020/069383 A1 offenbart eine Treiberschnittstelle für einen Roboterarm eines chirurgischen Robotersystem mit einem Antriebselement, einen Aktuator, ein Gehäuse mit einer Öffnung für das Antriebselement und eine Steriltuch-Erfassungsöffnung.

### Kurzbeschreibung der Offenbarung

Es ist also die Aufgabe der Offenbarung, die Nachteile aus dem Stand der Technik zu vermeiden oder wenigstens zu mildern. Insbesondere soll eine, zum Beispiel elektro-mechanische Schnittstelle an einem Operationstisch zur wiederholten Adaption/Trennung steriler Anwendungsteile an unsterile Betriebsteile sicher, hygienisch und/oder einfach damit kostengünstig sein.

Diese Aufgabe wird offenbarungsgemäß durch eine Sterilbarriere mit den Merkmalen des Anspruchs 1 gelöst. Vorteilhafte Weiterbildungen der Offenbarung sind Gegenstand der Unteransprüche.

Der Kerngedanke der vorliegenden Offenbarung besteht im Wesentlichen darin, ein einfaches Steriltrennungselement (Sterilbarriere) beispielsweise in Form einer Plane, Folie oder eines Tuchs sowie eine elektro-mechanische Schleuse/Kupplung vorzusehen, mit wenigstens einem Kontaktstift, der so angeordnet und ausgebildet ist, dass er nach Art eines Dorns in das Steriltrennungselement von dessen einer Seite aus eingesteckt/eingestochen werden kann/eingestochen ist, derart, dass er aus der anderen Seite des Steriltrennungslements vorragt. Dadurch wird erreicht, dass der zumindest eine Kontaktstift (unmittelbar/direkt) keimdicht vom Steriltrennungselement umgeben wird, bzw. das Steriltrennungselement keimdicht an dem zumindest einen Kontaktstift ringsherum anliegt, wobei der Kontaktstift an zumindest einem Ende/Endabschnitt von einer Leitung beispielsweise mittels einer Steckbuchse oder einem Stecker kontaktiert werden kann.

Bereits an dieser Stelle sei darauf hingewiesen, dass der zumindest eine Kontaktstift zwei zueinander im Wesentlichen gleichförmige/identische Stab-/Stiftenden aufweisen kann, derart, dass zwei zueinander gleichförmige/identische Steckdosen mit oder bestehend aus entsprechend buchsen-/hülsenförmigen Kontaktelementen beidseits aufgesteckt werden können. Alternativ hierzu ist es aber auch denkbar, den zumindest einen Kontaktstift mit (zwei) zueinander unterschiedlichen Stiftenden auszubilden, insbesondere derart, dass das eine Ende, vorzugsweise auf der unsterilen Seite des Steriltrennungselements, als (kleindurchmessriger) Stift und das andere Ende, vorzugsweise auf der sterilen Seite des Steriltrennungselements als (großdurchmessrige) Hülse/Steckbuchse geformt ist. Auf diese Weise kann auf der einen (unsterilen) Seite eine Art Steckdose/Steckbuchse mit (großdurchmessrigem) hülsenförmigem Kontaktstift und auf der anderen (sterilen) Seite eine Art Stecker/Einsteckstift mit (kleindurchmessriger) Stiftform in/auf den das Steriltrennungselement durchdringenden Kontaktstift auf-/eingesteckt werden. Letzteres bietet den Vorteil, dass beispielsweise elektrische medizinische Instrumente auf der sterilen Seite, welche für gewöhnlich mit elektrischen Kabeln und daran fest angeordneten Kabelsteckern (mit stiftförmigen elektrischen Kontakten) ausgerüstet sind, ohne zusätzliche (Zwischen-)Adapter mit dem zumindest einen Kontaktstift (bzw. dessen hülsenförmigem Stiftende) elektrisch gekoppelt werden können. Gleiches gilt auch für sogenannte Verlängerungskabel auf der unsterilen Seite, deren jeweils eine endseitige Steckdose mit zumindest einem hülsenförmigen Kontaktstift ebenfalls adapterlos mit den dort als Stift ausgeformten Stiftende des zumindest einen, in das Steriltrennungselement eingesetzten Kontaktstifts elektrisch gekoppelt werden können.

Vorzugsweise (alternativ oder kumulativ) ist der zumindest eine Kontaktstift auf der einen Seite des Steriltrennungselements in/durch einem/ein ersten(s) Gehäuse gehalten, welches entweder einen Aufnahmeschacht für eine Steckbuchse und/oder Stecker bildet oder aus welchem zumindest eine Leitung herausgeführt ist, die an dem zumindest einen Konstaktstift fixiert ist. Auch hier sei darauf hingewiesen, dass nicht notwendiger Weise auf den beiden Seiten des Steriltrennungselements eine gleiche Art von Aufnahmeschacht oder Steckbuchse angeordnet sein muss, sondern dass auch vorgesehen sein kann, auf der einen, vorzugsweise unsterilen Seite des Steriltrennungselements einen Stecker und auf der anderen, vorzugsweise sterilen Seite eine Steckbuchse anzuordnen.

Weiter vorzugsweise (alternativ oder kumulativ) ist auf der anderen Seite des Steriltrennungselements ein zweites Gehäuse vorgesehen, das dafür ausgebildet ist, über das Steriltrennungselement (als sterile Zwischenschicht) gegen das erste Gehäuse gedrückt zu werden, wobei dadurch der zumindest eine Kontaktstift das Steriltrennungselement durchsticht und in das Innere des zweiten Gehäuses ragt. Das zweite Gehäuse kann dabei bevorzugt als Aufnahmeschacht ausgebildet sein, derart, dass eine weitere Steckbuchse oder ein Stecker in das zweite Gehäuse eingesteckt und mit dem zumindest einen Kontaktstift kontaktiert werden kann; und weiter bevorzugt das zweite Gehäuse am Kontaktstift und damit am ersten Gehäuse hält.

Eine solche Ausgestaltung ist besonders einfach, preisgünstig und trotzdem sicher und damit besonders geeignet für Ein-Weg Sterilplanen.

In anderen Worten ausgedrückt, wird offenbarungsgemäß ein flächiges, vorzugsweise flexibles Sterilelement zur Steriltrennung bereitgestellt. Das Steriltrennungselement ist vorgesehen oder ausgebildet, zwischen ersten und zweiten Steckverbindern/Gehäusen einer Steckverbindung/elektro-mechanischen Schleuse angeordnet zu sein oder dazwischen eingepasst zu werden; und ist damit vorgesehen oder ausgebildet, eine Steriltrennung der Steckverbinder zu schaffen bzw. zu bewirken.

Die Steckverbindung kann vorzugsweise eine kraftschlüssige und/oder formschlüssige Verbindung sein. Beim Verbinden der ersten und zweiten Steckverbinder/Gehäuse kann sich vorzugsweise das Steriltrennnungsement zwischen den Steckverbindern befinden, so dass sich das Steriltrennungselement quasi selbsttätig an eine geometrische Form der Steckverbindung zwischen den Steckverbindern anpasst.

Unter (dem Begriff der) Steriltrennung kann hierin insbesondere verstanden werden, dass sterile Elemente von unsterilen Elementen so getrennt werden, dass das sterile Element zumindest an vorgesehenen Berührungspunkten bzw. zur händischen Bedienung/Berührung vorgesehenen Stellen nicht kontaminiert wird.

Vorteilhafte Ausführungsformen sind in den Unteransprüchen beansprucht und werden nachfolgend näher erläutert.

Zum Beispiel kann das Steriltrennungselement faltbar und/oder flexibel sein. Ebenfalls kann das Steriltrennungselement zur Einmalverwendung (englisch: single-use) vorgesehen sein, zum Beispiel als ein Wegwerfartikel. Hierdurch kann auf eine einfache Art und Weise ein sicheres und hygienisches Steriltrennungsmittel bereitgestellt werden.

Die Steckverbindung kann eine elektrische Steckverbindung sein. Einer der Steckverbinder der Steckverbindung kann männlich sein (Kontaktstift), und der andere der Steckverbinder der Steckverbindung kann weiblich sein (Steckbuchse).

Das Steriltrennungselement ist dafür vorgesehen, von einer Anzahl (oder Mehrzahl) elektrischer Kontakte des männlichen Steckverbinders (Kontaktstifte) durchdrungen zu werden. Das Steriltrennungselement kann insbesondere vorgesehen sein, zwischen dem weiblichen Steckverbinder/Steckbuchse und dem männlichen Steckverbinder (Kontaktstift) beim Steckvorgang derselben, eine Zwischenschicht zwischen dem weiblichen Steckverbinder und dem männlichen Steckverbinder auszubilden.

In einer vorteilhaften Ausgestaltung (alternativ oder kumulativ) kann das Steriltrennungselement so dimensioniert sein, dass das flächige Element bei Benutzung eine Stulpe über einen der Steckverbinder ausbildet. Hierdurch kann der Steckverbinder einfach gegriffen werden, ohne die Gefahr der Kontamination.

Das Steriltrennungselement kann beispielsweise (alternativ oder kumulativ) mit einem Desinfektionsmittel getränkt sein. Beispielsweise kann das Desinfektionsmittel auf Basis von Alkohol (Ethanol bzw. 2-Propanol) und Mecetroniumetilsulfat hergestellt sein. Zusätzlich kann das Desinfektionsmittel eine rückfettende Substanz enthalten, um keine Hautirritationen hervorzurufen. Somit kann das flächige Element ein Handdesinfektionstuch sein. Das Desinfektionsmittels kann ebenfalls ein oder mehrere der folgenden Mittel enthalten: Oxidationsmittel, Peressigsäure, Wasserstoffperoxid, Halogene (Iod-, Chlorverbindungen), Ozon, Phenolderivate (u.a. Chlorxylenol, Triclosan), Aldehyde (z.B. Formaldehyd), Alkohole, Detergentien, Stickstoffverbindungen (z.B. Benzalkoniumchlorid) und dergleichen (Chlorhexidin, Octenidin, Polyhexanid). Somit kann das flächige Element selbst steril/keimfrei sein.

Ebenfalls kann das Steriltrennungselement vorzugsweise (alternativ oder kumulativ) ausgelegt sein, bei Benutzung, die Stulpe bis zu einem an dem Steckverbinder angeschlossenen Kabel zu bilden. Hierdurch kann dem Personal ein leichtes Bedienen/Anschließen der Steckverbinder ermöglicht werden, ohne sich zu kontaminieren.

Gemäß einer oder mehrerer Ausführungsformen kann das Steriltrennungselement eine Folie, ein Tuch oder eine Plane sein. Das Steriltrennungselement kann dabei reißfest ausgebildet sein. Zumindest kann das flächige Element eine vorbestimmte Zugkraft, zum Beispiel mindestens 1 N/mm² oder 10 N/mm² aushalten, also zugfest sein. Ebenfalls kann das flächige Element auf eine maximale Zugkraft ausgelegt sein, zum Beispiel 15 N/mm² oder 150 N/mm².

Der unsterile Steckverbinder kann insbesondere (alternativ oder kumulativ) eine Befestigung zur Anbringung an einen externen ortsfesten Gegenstand wie einen Operationstisch aufweisen. Hierdurch kann dem Personal ermöglicht werden, lediglich sterile Teile anfassen zu müssen, um das sterile und das unsterile Kabel, zum Beispiel mittels des Steckverbindungsadapters oder mittels des flächigen Elements, zu verbinden.

### Kurzbeschreibung der Figuren

Die Offenbarung wird nachfolgend anhand eines bevorzugten Ausführungsbeispiels unter Bezugnahme auf die begleitenden Figuren näher erläutert. Es zeigen:

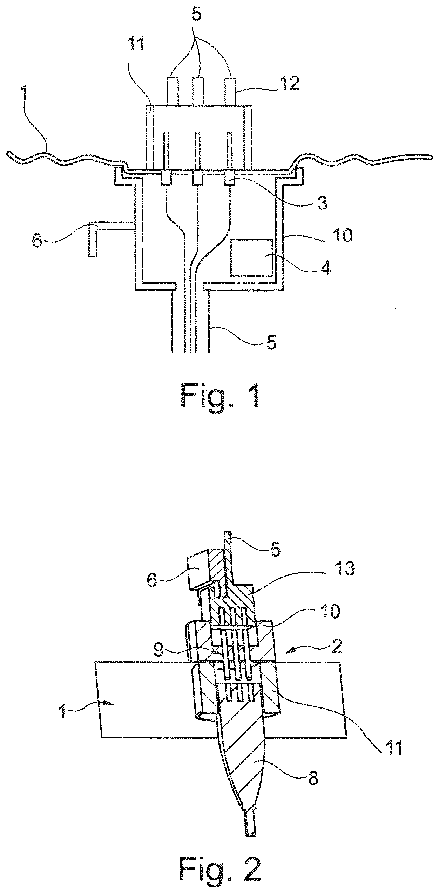

- Figur 1: eine schematische Darstellung einer Sterilbarriere in Form eines Steriltrennungselements mit einer elektro-mechanischen Schleuse in allgemeiner Darstellung bzw. in einem allgemeinen Grundaufbau zur Veranschaulichung wesentlicher Bauteile / Komponenten einer offenbarungsgemäßen Anordnung,
- Figur 2: eine schematische Darstellung einer Sterilbarriere in Form eines Steriltrennungselements mit der elektro-mechanischen Schleuse gemäß einem ersten bevorzugten Ausführungsbeispiel der vorliegenden Offenbarung (betreffs des zwei freie Stiftenden aufweisenden zumindest einen elektrischen Kontaktstifts), insb. ausgebildet als ein Steriltrennungsmodul;
- Fig. 3: eine schematische Darstellung einer Sterilbarriere in Form eines Steriltrennungselements mit der elektro-mechanischen Schleuse gemäß einem zweiten bevorzugten Ausführungsbeispiel der vorliegenden Offenbarung (betreffs des zwei freie Stiftenden aufweisenden zumindest einen elektrischen Kontaktstifts), insb. ausgebildet als ein Steriltrennungsmodul und

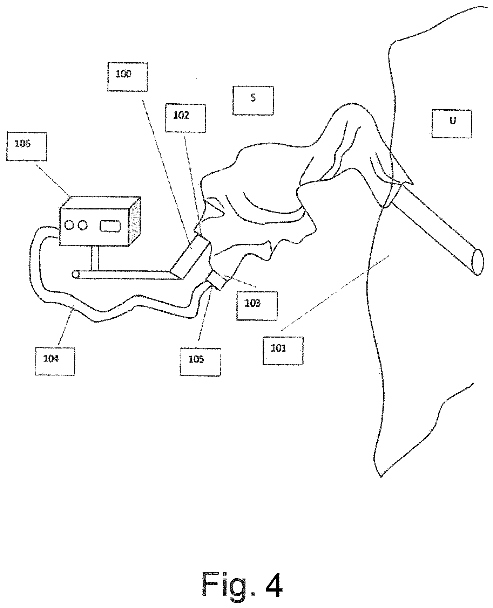

- Fig. 4: eine allgemeine Anordnung/Unterteilung eines sterilen und unsterilen Bereichs.

### Figurenbeschreibung

Fig. 1 zeigt eine Sterilbarriere in allgemeiner Darstellung bzw. in einem allgemeinen Grundaufbau zur Veranschaulichung wesentlicher Bauteile / Komponenten einer offenbarungsgemäßen Anordnung, wobei die Anordnung aufweist:
- ein flexibles, vorzugsweise tuch- oder planenartiges Steriltrennungselement 1 (nachfolgend einfach als Steriltuch bezeichnet);
- eine elektro-mechanische Schleuse 2 im/am Steriltuch 1, aufweisend zumindest einen elektrischen Kontaktstift 9, der das Steriltrennungselement 1 vereinzelt durchdringt; und
- einen (ersten) Leitungsbus 5, bestehend aus einer Anzahl/Mehrzahl an Leitungen/Kabeln, der in einem Unsterilbereich zur Schleuse 2 hinführt, und einen weiteren (zweiten) Leitungsbus 5, bestehend aus einer entsprechenden Anzahl/Mehrzahl an Leitungen/Kabeln, der im Sterilbereich von der Schleuse 2 wegführt.

Im Konkreten ist das Steriltuch 1 ein keimdichtes Gewebe oder eine (keimdichte) Folie, das bzw. die dafür vorgesehen und ausgebildet ist, einen Sterilbereich von einem Unsterilbereich zu trennen. Beispielsweise kann der Sterilbereich ein OP-Bereich/Raum sein, in welchem sich ein Robotorarm oder diverse Antriebs- und/oder Steuerungseinrichtungen/medizinische Geräte befinden, die für sich betrachtet Unsterilbereiche darstellen und daher vom Sterilbereich keimdicht isoliert werden müssen. Zu diesem Zweck können derartige Unsterilbereiche von dem dargestellten Steriltuch 1 umgeben/ummantelt werden, wobei jedoch gewährleistet sein muss, dass z.B. elektrische Geräte, Energiequellen oder Steuerungen innerhalb des Unsterilbereichs durch das Steriltuch 1 hindurch elektrischen Kontakt zu (medizintechnischen bzw. chirurgischen) Geräten, wie z.B. chirurgischen elektromotorischen Handgriffen, Beleuchtungsmitteln, etc. im Sterilbereich haben, ohne dass die keimdichte Sterilbarriere beschädigt/unterbrochen wird.

Dieser grundsätzliche Aufbau bzw. allgemeine Grundaufbau entspricht jener Anordnung gemäß der Fig. 4, sodass hinsichtlich dieser auf die vorstehende Beschreibung unter Bezugnahme auf die Fig. 1 verwiesen werden kann. Zum Zweck der Aufrechterhaltung der Sterilität ist die offenbarungsgemäße elektro-mechanische Schleuse 2 vorgesehen, wie sie nachfolgend näher beschrieben wird.

Grundsätzlich hat die elektro-mechanische Schleuse 2 gemäß der vorliegenden Offenbarung eine Anzahl, vorzugsweise eine Mehrzahl von Kontaktstiften (Schleusenkontakten) 9, die bevorzugt in einem ersten Gehäuse (Muffe) 10 gelagert/fixiert sind, derart, dass sie an einer Gehäuseseite (nachfolgend als Anlageseite bezeichnet) aus dem ersten Gehäuse 10 dornförmig hervorragen. Die Kontaktstifte 9 sind dabei in einem Parallelabstand zueinander angeordnet.

Jeder Kontaktstift 9 hat zumindest ein freies/frei vorragendes Stiftende bzw. einen freien Endabschnitt, der (bzw. das) dafür vorgesehen und ausgebildet/angepasst ist, das Steriltuch 1 zu durchstechen. Zu diesem Zweck kann das eine Stiftende des entsprechenden Kontaktstifts 9 beispielsweise angespitzt oder angeschärft sein, oder der betreffende Kontaktstift 9 hat einen so geringen Durchmesser, dass dieser durch das Steriltuch 1 gestochen werden kann, ohne dass das Steriltuch 1 ausreißt. Entscheidend hierbei ist, dass ein sehr enges Einstechloch gebildet wird, sodass der eingestochende Kontaktstift 9 anschließend keimdicht vom Steriltuch 1 vollumfänglich anliegend umgeben ist.

Vorzugsweise kann sich bereits innerhalb des ersten Gehäuses 10 Steuerungselektronik befinden, beispielsweise eine Elektronik 4 zur Steuerung des Akkus eines elektrischen medizintechnischen/chirurgischen Geräts, das sich innerhalb des Sterilbereichs befindet, oder von dergleichen Arbeitsgerätschaften. In diesem Fall ist jedem elektrischen medizintechnischen/chirurgischen Gerät innerhalb des Sterilbereichs eine gerätespezifische Schleuse 2 zugeordnet, beispielsweise mit gerätespezifischen Steuerungselementen oder Energieversorgungen oder dergleichen bereits innerhalb des ersten Schleusen-Gehäuses 10.

Nach dem weiteren offenbarungsgemäßen Teilaspekt gemäß der Fig. 1 hat der zumindest eine elektrische Kontaktstift 9 ein freies Stiftende, das dafür vorgesehen und ausgebildet ist, mit einer entsprechenden elektrischen Steckbuchse 12 der wenigstens einen Leitung 5 auf der sterilen oder unsterilen Seite des Steriltrennungselements 1 in elektro-mechanischen oder elektrisch leitenden Steckkontakt zu kommen, wohingegen am anderen Stiftende die wenigstens andere Leitung 5 auf der entsprechend anderen Seite des Steriltrennungselements 1 fest angeschlossen ist. Dabei ist an das andere freie Stiftendebzw. den anderen freien Endabschnitt jedes Kontaktstifts 9 eine Leitung (Kabel) fixiert, die zu einem Leitungsbus (Kabelbaum) 5 zusammengefasst sind, der durch eine Gehäuseöffnung an einer der Anlageseite abgewandten Gehäuseseite nach außen geführt ist. Dieser Kabelbaum 5 ist dann innerhalb des Sterilbereichs mit einem entsprechenden medizinischen Gerät elektrisch gekoppelt (nicht weiter dargestellt).

Schließlich kann allgemein (wie anhand Figur 1 dargestellt) an dem ersten Gehäuse 10 eine Halterung 6 angeordnet/ausgebildet sein, mit welcher das erste Gehäuse 10 an einem (orts-)festen Gegenstand, beispielsweise einem OP-Tisch fixiert werden kann, um so die Schleuse 2 und auch das Steriltuch 1 ortsfest zu halten.

Auf der dem ersten Gehäuse 10 abgewandten Seite des Steriltuchs 1 ist ein zweites Gehäuse 11 gezeigt, welches eine im wesentlichen hohlzylindrische/rohrartige Form aufweist, wobei dessen eine Stirnseite am Steriltuch anliegt, und wobei das Steriltuch 1 zwischen dem ersten 10 und dem zweiten Gehäuse 11 sandwichartig zu liegen kommt. Dabei ragen die einen freien Stiftenden/Endabschnitte der Kontaktstifte 9 (welche durch das Steriltuch 1 bereits hindurchgedrückt wurden) in den Innenraum des hohlzylindrischen zweiten Gehäuses 11 hinein.

Diese freien Stiftenden der Kontaktstifte 9 sind dafür vorgesehen und ausgebildet, mit Kontaktbuchsen 12 in elektrischen Steckkontakt zu kommen, an denen ebenfalls Leitungen/Kabel befestigt sind, die zu einem Kabelbaum 5 vergleichbar zu dem Kabelbaum 5 auf der anderen Steriltuchseite zusammengeführt sind. Sobald diese Steckbuchsen 12 auf die freien Stiftenden/Endabschnitte der Kontaktstifte 9 aufgesteckt sind, drücken sie das Steriltuch 1 gegen die Anlageseite des auf der anderen Steriltuchseite liegenden ersten Gehäuses 10 und halten somit den gesamten Schleusenaufbau zusammen.

An dieser Stelle sei darauf hingewiesen, dass die in Fig. 1 vereinzelt dargestellten Kontaktbuchsen 12 auch zu einem Stecker zusammengefasst sein können, dessen Form an das hohlzylindrische zweite Gehäuse 12 angepasst ist, um in dieses eingesteckt werden zu können.

Die Fig. 2 zeigt ein erstes Ausführungsbeispiel der vorliegenden Offenbarung, wobei im folgenden im Wesentlichen auf die zum Beispiel gemäß der Fig. 1 unterschiedlichen Konstruktionsmerkmale eingegangen wird (alle anderen vorstehenden Beschreibungen gelten damit auch für das offenbarungsgemäße erste Ausführungsbeispiel); dabei werden für gleiche Bauteile auch die gleichen Begriffe und Bezugszeichen verwendet.

In diesem Fall bildet das erste Gehäuse 10 im Unterschied zu dem ersten Gehäuse 10 gemäß der Fig. 1 (in Übereinstimmung mit dem zweiten Gehäuse 11) ebenfalls einen Einsteckschacht für eine Anzahl an Steckbuchsen oder einen Stecker 13, in welchem die Steckbuchsen zusammengefasst sind und welcher an den Einsteckschacht des ersten Gehäuses 10 angepasst ist. D.h. in dem offenbarungsgemäßen ersten Ausführungsbeispiel (bzw. der Abwandlung) gemäß der Fig. 2 sind die festen Leitungsverbindungen 3, wie sie in der Fig. 1 gezeigt sind, durch eine weitere Steckverbindung ersetzt, sodass die Kontaktstifte 9 in diesem Fall zwei freie Stiftenden/Endabschnitte haben, die für eine Steckverbindung mit entsprechenden Leitungen/Kabeln vorgesehen und ausgebildet sind.

Des Weiteren ist in dem offenbarungsgemäßen ersten Ausführungsbeispiel gemäß der Fig. 2 die Halterung 6 zur Fixierung der Schleuse 2 an einem (orts-)festen Gegenstand nicht am ersten Gehäuse 10, sondern an dem Stecker 13 angeordnet/ausgebildet. Im Übrigen ist in dem offenbarungsgemäßen ersten Ausführungsbeispiel gemäß der Fig. 2 auch jener Stecker 8 nunmehr bildlich dargestellt, wie er zuvor als Ersatz/Alternative für die in Fig. 1 gezeigten Steckbuchsen 12 erwähnt wurde.

Die Offenbarung betrifft zusammengefasst ein Steriltrennungselement 1 zur Steriltrennung, das vorgesehen ist, zwischen Steckverbindern 10, 11 einer Steckverbindung/elektro-mechanischen Schleuse angeordnet zu sein und damit eine Steriltrennung der Steckverbinder 10, 11 bereitzustellen.

In Fig. 3 ist ein zweites Ausführungsbeispiel der vorliegenden Offenbarung zumindest schematisch gezeigt, wobei nachfolgend nur die Unterschiede zu dem ersten Ausführungsbeispiel konkreter beschrieben werden.

Wie in der Fig. 2 zu sehen ist, sind die Kontaktstifte 9 als gleichförmige Stäbe ausgebildet, die auf beiden Seiten des Steriltrennungselements 1 Einsteckenden bilden, die nach Art eines Steckers in Einsteckbuchsen nach Art einer Steckdose eingesteckt werden können. Dies bedeutet, dass an beiden Seiten des Steriltrennungselements 1 Steckdosen vorgesehen sein müssen, die mit beidseits ausgebildeten Steckern der elektrischen/elektro-mechanischen Schleuse 2 in Kontakteingriff gebracht werden können.

Es hat sich aber gezeigt, dass medizinische elektrische Instrumente bereits mit elektrischen Kabeln ausgerüstet sind, an denen in der Regel bereits Stecker mit einfachen Kontaktstiften montiert sind. Um diese an der Schleuse 2 gemäß der Fig. 2 anzuschließen, müsste ein zusätzlicher Adapter vorgesehen sein. Zur Vermeidung eines solchen zusätzlichen Bauteils ist es im zweiten Ausführungsbeispiel der Offenbarung vorgesehen, zumindest an der sterilen Seite des Steriltrennungselements 1 den wenigstens einen Kontaktstift 9 endseitig hülsen-/steckbuchsenförmig zu gestalten, derart, dass die Schleuse 2 auf der Sterilseite des Steriltrennungselements 1 eine Art Steckdose bildet, in welche dann ein Stecker mit zumindest einem einzigen, einfachen Kontaktstift einsteckbar ist, wie dieser beispielsweise in der Fig. 1 gezeigt ist. Diese Variante hat gegenüber der Variante gemäß Fig. 2 den Vorteil, dass ein am medizinische Instrument bereits vorhandener Stecker unmittelbar mit der Schleuse 2 in Stecckontakt bringbar ist.

### Bezugszeichenliste

- 1: Steriltrennungselement/Steriltuch/Drape
- 2: Elektro-mechanische Schleuse/Steriltrennungsmodul
- 3: Feste Leitungsverbindungen/Kontakte
- 4: Elektronik
- 5: Kabelbaum/Leitungsbus
- 6: Halterung
- 8, 13: Stecker
- 9: Kontaktstifte/Schleusenkontakte
- 10: Erstes Gehäuse/Steckverbinder
- 11: Zweites Gehäuse/Steckverbinder
- 12: Steckbuchsen

## Patentansprüche

1. Sterilbarriere mit einem flächigen Steriltrennungselement (1) in Form einer Abdeck- oder Abtrennplane, und einer im/am Steriltrennnungselement (1) ein-/ansetzbaren oder ein-/angesetzten elektro-mechanischen Schleuse (2) zur elektrischen Verbindung zumindest einer elektrischen Leitung (5) auf der einen, unsterilen Seite des Steriltrennungselements (1) mit zumindest einer elektrischen Leitung (5) auf der anderen, sterilen Seite des Steriltrennungselements (1), wobei die elektro-mechanische Schleuse (2) zumindest einen elektrischen Kontaktstift (9) hat, wobei der zumindest eine elektrische Kontaktstift (9) zwei frei vorragende Stiftenden hat, die dafür vorgesehen und ausgebildet sind, mit entsprechenden elektrischen Stiften und/oder Steckbuchsen (12) der wenigstens zwei zu verbindenden Leitungen (5) in elektro-mechanischen oder elektrisch leitenden Steckkontakt zu kommen, **dadurch gekennzeichnet, dass** der zumindest eine elektrische Kontaktstift (9) beim Ein-/Ansetzen der elektro-mechanischen Schleuse (2) im/am Steriltrennungselement (1) das Steriltrennungselement (1) vereinzelt einstechend durchdringt, derart, dass das Steriltrennungselement (1) unmittelbar am zumindest einen in dieses eingestochenen elektrischen Kontaktstift (9) keimdicht vollumfänglich anliegt.

2. Sterilbarriere nach Anspruch 1, **dadurch gekennzeichnet, dass** das Steriltrennungselement (1) folien-, tuch- und/oder plattenartig ist.

3. Sterilbarriere nach einem der vorstehenden Ansprüche 1 bis 2, **dadurch gekennzeichnet, dass** die elektro-mechanische Schleuse (2) ein elektro-mechanisches Kopplungsmodul bildet, mit zumindest einem ersten Modulgehäuse (10), das eine Steriltrennungselement-Anlageseite oder Anlagefläche hat, aus welcher das eine freie Stiftende des zumindest einen elektrischen Kontaktstifts (9) frei vorragt.

4. Sterilbarriere nach Anspruch 3, **dadurch gekennzeichnet, dass** das erste Gehäuse auf der der Anlageseite abgewandten Gehäuseseite einen Einsteckschacht bildet, in welchen das andere freie Stiftende des zumindest einen elektrischen Kontaktstifts (9) hineinragt und in welchen die zumindest eine Steckbuchse (12) einsteckbar ist.

5. Sterilbarriere nach einem der vorstehenden Ansprüche 3 bis 4, **dadurch gekennzeichnet, dass** das elektro-mechanische Kopplungsmodul ein zweites Modulgehäuse (11) in Form eines hohlen Zylinders hat, der einen axial durchgehenden Einsteckschacht umschließt und der dafür vorgesehen und ausgebildet ist, an einer Stirnseite gegen das Steriltrennungselement (1) angelegt zu werden, derart, dass das freie Ende des zumindest einen, das Steriltrennungselement (1) bereits durchdrungenen Kontaktstifts (9) in den Einsteckschacht des zweiten Modulgehäuses (11) ragt wobei auf der anderen Stirnseite die Steckbuchse (12) der zumindest anderen Leitung (5) einsteckbar ist.

6. Sterilbarriere nach einem der vorstehenden Ansprüche 1 bis 5, **dadurch gekennzeichnet, dass** die Stiftenden oder Stiftendabschnitte auf einer der Seiten, insbesondere auf der unsterilen Seite, einen männlichen Stecker ausbilden, der dafür ausgebildet und ausgelegt ist, mit einer weiblichen Steckbuchse eines Kabels verbunden zu werden.

7. Sterilbarriere nach einem der vorstehenden Ansprüche 1 bis 6, **dadurch gekennzeichnet, dass** die Stiftenden oder Stiftendabschnitte auf einer der Seiten, insbesondere auf der sterilen Seite, eine weibliche Steckbuchse ausbilden, die dazu vorgesehen ist, mit einem männlichen Stecker eines Kabels verbunden zu werden.

8. Sterilbarriere nach den Ansprüchen 6 und 7, **dadurch gekennzeichnet, dass** der männliche Stecker und die weibliche Steckbuchse komplementär ausbildet sind.

9. Sterilbarriere nach einem der vorstehenden Ansprüche 1 bis 8, **dadurch gekennzeichnet, dass** eine Mehrzahl von Kontaktstiften (9) vorgesehen ist, die parallelbeabstandet sind, wobei eine entsprechende Anzahl an Kontaktbuchsen (12) auf zumindest einer Seite des Steriltrennungselements (1) vorgesehen ist, die vorzugsweise zu einem einzigen Stecker (8, 13) zusammengefasst sind.

10. Sterilbarriere nach Anspruch 9, **dadurch gekennzeichnet, dass** das erste Modulgehäuse (10) oder der Stecker (13) mit einer Halterung (6) ausgebildet oder mit dieser ausrüstbar ist, die dafür vorgesehen und ausgebildet ist, an einem externen Gegenstand insbesondere einem OP-Tisch fixiert zu werden.

11. System mit einem medizinischen Instrument, einer Energiequelle für das medizinische Instrument und einer Sterilbarriere nach einem der vorhergehenden Ansprüche 1 bis 10, wobei die elektro-mechanische Schleuse (2) der Sterilbarriere in Form eines Zwischensteckers zwischen dem medizinischen Instrument und der Energiequelle, vorzugsweise in einem planen- oder folienartigen Steriltrennungselement (1), angeordnet ist.

## Claims

1. Sterility barrier with a flat sterility separation element (1) in form of a covering or separation sheet, and an electro-mechanical lock (2), which is insertable/positionable or inserted/positioned in/on the sterility separation element (1), for the electrical connection of at least one electrical line (5) on the one, non-sterile side of the sterility separation element (1) to at least one electrical line (5) on the other, sterile side of the sterility separation element (1), the electro-mechanical lock (2) having at least one electrical contact pin (9), wherein
the at least one electrical contact pin (9) has two freely projecting pin ends which are provided and configured to come into electro-mechanical or electroconductive plug contact with corresponding electrical pins and/or plug sockets (12) of the at least two lines (5) to be connected,
**characterized in that**
the at least one electrical contact pin (9) individually penetratingly enters the sterility separation element (1) when inserting/positioning the electro-mechanical lock (2) in/on the sterility separation element (1), such that the sterility separation element (1) fits directly to the at least one electrical contact pin (9) penetrating it, over its entire circumference in a germ-tight manner.

2. Sterility barrier according to claim 1, **characterized in that** the sterility separation element (1) is foil-like, drape-like, and/or plate-like.

3. Sterility barrier according to any of the preceding claims 1 to 2, **characterized in that** the electro-mechanical lock (2) forms an electro-mechanical coupling module with at least one first module housing (10) having a sterility separation element abutment side or abutment surface from which the one free pin end of the at least one electrical contact pin (9) projects freely.

4. Sterility barrier according to claim 3, **characterized in that** the first housing forms, on the housing side facing away from the abutment side, an insertion shaft into which the other free pin end of the at least one electrical contact pin (9) projects and into which the at least one plug socket (12) is insertable.

5. Sterility barrier according to any of the preceding claims 3 to 4, **characterized in that** the electro-mechanical coupling module has a second module housing (11) in form of a hollow cylinder which encloses an axially continuous insertion shaft and which is provided and configured to be abutted to the sterility separation element (1) on one end face such that the free end of the at least one contact pin (9), that has already penetrated the sterility separation element (1), projects into the insertion shaft of the second module housing (11), wherein, on the other end face, the plug socket (12) of the at least one other line (5) is insertable.

6. Sterility barrier according to any of the preceding claims 1 to 5, **characterized in that** the pin ends or pin end sections on one of the sides, in particular on the non-sterile side, form a male plug configured and adapted to be connected to a female plug socket of a cable.

7. Sterility barrier according to any of the preceding claims 1 to 6, **characterized in that** the pin ends or pin end sections on one of the sides, in particular on the sterile side, form a female plug socket provided to be connected to a male plug of a cable.

8. Sterility barrier according to claims 6 and 7, **characterized in that** the male plug and the female plug socket are of complementary form.

9. Sterility barrier according to any of the preceding claims 1 to 8, **characterized in that** a plurality of contact pins (9) is provided which are spaced apart in parallel, wherein a corresponding number of contact sockets (12) is provided on at least one side of the sterility separation element (1), which are preferably combined to form a single plug (8, 13).

10. Sterility barrier according to claim 9, **characterized in that** the first module housing (10) or the plug (13) is configured or equippable with a holder (6) which is provided and configured to be fixed to an external object, in particular an operating table.

11. System with a medical instrument, an energy source for the medical instrument and a sterility barrier according to any of the preceding claims 1 to 10, wherein the electro-mechanical lock (2) of the sterility barrier arranged in form of an intermediate plug between the medical instrument and the energy source, preferably in a sheet-like or foil-like sterility separation element (1).

## Revendications

1. Barrière stérile avec un élément de séparation stérile (1) plan sous la forme d'un plan de recouvrement ou de séparation, et un sas (2) électromécanique inséré/placé ou pouvant être inséré/placé dans/au niveau de l'élément de séparation stérile (1) pour la liaison électrique au moins d'une conduite électrique (5) sur l'un côté non stérile de l'élément de séparation stérile (1) avec au moins une conduite électrique (5) sur l'autre côté stérile de l'élément de séparation stérile (1), dans laquelle le sas (2) électromécanique présente au moins une tige de contact (9) électrique,
dans laquelle l'au moins une tige de contact (9) électrique présente deux extrémités de tige en saillie libre qui sont prévues et formées afin de venir en contact d'enfichage électromécanique ou électroconducteur avec des tiges et/ou douilles d'enfichage (12) électriques correspondantes des au moins deux conduites (5) à relier,
**caractérisée en ce que**
l'au moins une tige de contact (9) électrique traverse sporadiquement par perçage lors de l'insertion/du placement du sas (2) électromécanique dans/au niveau de l'élément de séparation stérile (1) l'élément de séparation stérile (1) de telle manière que l'élément de séparation stérile (1) repose sur toute la périphérie de manière étanche aux germes directement contre au moins une tige de contact (9) électrique percée dans celui-ci.

2. Barrière stérile selon la revendication 1, **caractérisée en ce que** l'élément de séparation stérile (1) est de type feuille, tissu et/ou plaque.

3. Barrière stérile selon l'une quelconque des revendications précédentes 1 à 2, **caractérisée en ce que** le sas (2) électromécanique forme un module de couplage électromécanique, avec au moins un premier boîtier de module (10) qui présente un côté d'appui d'élément de séparation stérile ou surface d'appui, de laquelle l'une extrémité de tige libre de l'au moins une tige de contact (9) électrique dépasse librement.

4. Barrière stérile selon la revendication 3, **caractérisée en ce que** le premier boîtier forme sur le côté de boîtier éloigné du côté d'appui un puits d'enfichage, dans lequel l'autre extrémité de tige libre de l'au moins une tige de contact (9) électrique pénètre et dans lequel l'au moins une douille d'enfichage (12) peut être enfichée.

5. Barrière stérile selon l'une quelconque des revendications précédentes 3 à 4, **caractérisée en ce que** le module de couplage électromécanique présente un second boîtier de module (11) sous la forme d'un cylindre creux, qui entoure un puits d'enfichage traversant axialement et qui est prévu et formé afin d'être posé contre un côté frontal contre l'élément de séparation stérile (1) de telle manière que l'extrémité libre de l'au moins une tige de contact (9) ayant déjà traversé l'élément de séparation stérile (1) pénètre dans le puits d'enfichage du second boîtier de module (11), dans laquelle la douille d'enfichage (12) de l'au moins autre conduite (5) peut être enfichée sur l'autre côté frontal.

6. Barrière stérile selon l'une quelconque des revendications précédentes 1 à 5, **caractérisée en ce que** les extrémités de tige ou sections d'extrémité de tige forment sur l'un des côtés, en particulier sur le côté non stérile, une fiche mâle qui est formée et conçue afin d'être reliée à une douille d'enfichage femelle d'un câble.

7. Barrière stérile selon l'une quelconque des revendications précédentes 1 à 6, **caractérisée en ce que** les extrémités de tige ou sections d'extrémité de tige forment sur l'un des côtés, en particulier sur le côté stérile, une douille d'enfichage femelle qui est prévue afin d'être reliée à une fiche mâle d'un câble.

8. Barrière stérile selon les revendications 6 et 7, **caractérisée en ce que** la fiche mâle et la douille d'enfichage femelle sont formées de manière complémentaire.

9. Barrière stérile selon l'une quelconque des revendications précédentes 1 à 8, **caractérisée en ce qu'**une pluralité de tiges de contact (9) est prévue, lesquelles sont espacées parallèlement, dans laquelle un nombre correspondant de douilles de contact (12) est prévu sur au moins un côté de l'élément de séparation stérile (1), lesquelles sont de préférence réunies en une seule fiche (8, 13).

10. Barrière stérile selon la revendication 9, **caractérisée en ce que** le premier boîtier de module (10) ou la fiche (13) est formée avec un support (6) ou peut être équipée de celui-ci, lequel est prévu et formé afin d'être fixé à un objet externe en particulier une table d'opération.

11. Système avec un instrument médical, une source d'énergie pour l'instrument médical et une barrière stérile selon l'une quelconque des revendications précédentes 1 à 10,
dans lequel le sas (2) électromécanique de la barrière stérile sous la forme d'une fiche intermédiaire est agencé entre l'instrument médical et la source d'énergie, de préférence dans un élément de séparation stérile (1) de type plan ou feuille.
